(19) Europäisches Patentamt
European Patent Office
Office européen des brevets

(11) EP 1 205 476 B1

(12) FASCICULE DE BREVET EUROPEEN

(45) Date de publication et mention de la délivrance du brevet:
**23.04.2003 Bulletin 2003/17**

(51) Int Cl.7: **C07D 317/60**, C07C 327/06, C07C 327/16

(21) Numéro de dépôt: **01402833.6**

(22) Date de dépôt: **31.10.2001**

# (54) Procédé de synthhèse de dérivés N-(mercaptoacyl)-amino-acides à partir d'acides acryliques alpha-substitués

Verfahren zu Synthese von N-(mercaptoacyl)-aminosäure Derivaten von alfasubstituierte Acrylsäuren an

Process for the synthesis of N-(mercaptoacyl)-amino acids derivatives from alpha-substituted acrylic acids

(84) Etats contractants désignés:
**AT BE CH CY DE DK ES FI FR GB GR IE IT LI LU MC NL PT SE TR**

(30) Priorité: **09.11.2000 FR 0014419**

(43) Date de publication de la demande:
**15.05.2002 Bulletin 2002/20**

(73) Titulaire: **BIOPROJET**
**75003 Paris (FR)**

(72) Inventeurs:
- **Monteil, Thierry**
  **76690 Saint Georges sur Fontaine (FR)**
- **Danvy, Denis**
  **76190 Yvetot (FR)**
- **Plaquevent, Jean-Christophe**
  **76960 Notre-Dame de Bondeville (FR)**
- **Duhamel, Pierre**
  **76130 Mont-Saint-Aignan (FR)**
- **Duhamel, Lucette**
  **76130 Mont-Saint-Aignan (FR)**
- **Lecomte, Jeanne-Marie**
  **75003 Paris (FR)**
- **Schwarts, Jean-Charles**
  **75014 Paris (FR)**
- **Piettre, Serge**
  **76270 Saint-Martin 1'Hortier (FR)**

(74) Mandataire: **Bernasconi, Jean Raymond et al**
**c/o Cabinet Lavoix,**
**2, Place d'Estienne d'Orves**
**75441 Paris Cedex 09 (FR)**

(56) Documents cités:
**EP-A- 0 419 327** **EP-A- 0 729 936**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 205 476 B1

## Description

La présente invention a trait à un procédé industriel de synthèse de dérivés N-(mercaptoacyl)-amino-acides à partir d'acides acryliques α-substitués.

Elle concerne plus particulièrement un nouveau procédé de synthèse de dérivés N-(mercaptoacyl)-amino-acides de formule générale (I) :

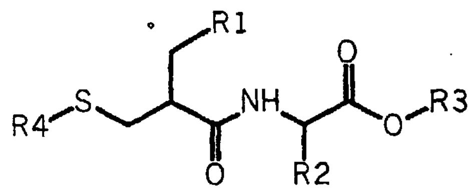

(I)

dans laquelle :

- R1 représente :
  - un groupe phényle ; ou
  - un groupe 3,4-méthylènedioxy-phényle ;
- R2 représente un atome d'hydrogène ou un groupe alkyle inférieur ;
- R3 représente un atome d'hydrogène, un groupe alkyle inférieur ou un groupe phénylalkylène inférieur ; et
- R4 représente un radical acyle aliphatique linéaire ou ramifié, ou un radical acyle aromatique.

Par "groupe alkyle inférieur" au sens de la présente invention, on entend, un groupe alkyle à chaîne(s) linéaire (s) ou ramifiée(s) contenant de 1 à 6 atomes de carbone, et de préférence de 1 à 4 atomes de carbone.

De même, au sens de l'invention, on entend par "groupe alkylène inférieur" un groupe alkylène contenant de 1 à 6 atomes de carbone, et de préférence de 1 à 4 atomes de carbone.

Les composés de formule (I) possèdent des propriétés pharmacologiques intéressantes. Ainsi, ils exercent notamment une activité inhibitrice sur certaines enzymes, telles que l'endopeptidase neutre (EC 3.4.24.11) et l'enzyme de conversion de l'angiotensine (EC 3.4.15.1). L'administration des composés de formules (I) permet donc de réduire ou de supprimer l'activité de ces enzymes, responsables respectivement de l'inactivation des enképhalines, du facteur atrial natriurétique, et de la transformation de l'angiotensine I en angiotensine II. En thérapeutique, ces composés exercent des activités antisecrétoires intestinales ou antihypertensives et sont utilisés dans le traitement de l'insuffisance cardiaque chronique. De plus, de tels composés peuvent être également utilisés dans le traitement de l'ostéoporose, comme cela a notamment été décrit dans la demande de brevet internationale WO 94/21242.

A titre de composés de formule (I) particulièrement intéressants, on peut citer les deux composés suivants :

1) le racécadotril (N-(RS)-[2-acétylthiométhyl-1-oxo-3-phényl-propyl]-glycinate de benzyle), de formule (II) :

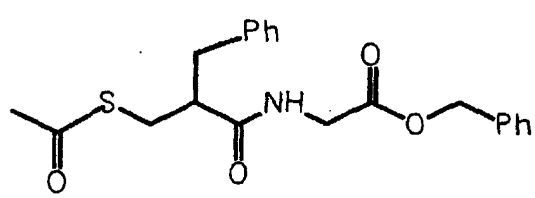

(II)

2) le fasidotril (N-(S)-[2-acétylthiométhyl-1-oxo-3-(3,4-méthylènedioxy-phényl)-propyl]-(S)-alaninate de benzyle), de formule (III) :

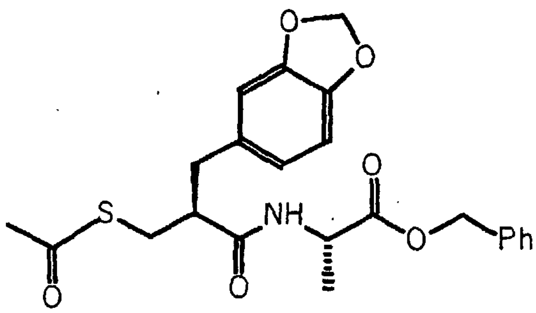

(III)

En ce qui concerne la préparation et l'utilisation de composés de formules (I) du type du racécadotril de formule (II), leur préparation et leur utilisation en thérapeutique, on pourra se reporter à la demande de brevet EP 0 038 758.

De même, on pourra se reporter à la demande de brevet EP 0 419 327 en ce qui concerne la préparation et les applications thérapeutiques des composés de formules (I) du type du fasidotril de formule (III).

De façon plus générale, un procédé de synthèse des composés de formule (I) à partir d'acides acryliques alpha-substitués est décrit dans la demande de brevet EP 0 729 936. Ce procédé fait-spécifiquement intervenir une première étape d'addition de Michaël d'un thioacide sur le groupement α-β-insaturé C=C-C=O de l'acide acrylique, puis une seconde étape de couplage peptidique d'un amino-ester sur le groupement -COOH en présence d'un agent de couplage tel que le DCC (dicyclohexylcarbodiimide).

Le procédé de synthèse décrit dans EP 0 729 936 est relativement efficace. Par ailleurs, il est intéressant du point de vue de son coût, notamment du fait qu'il met en oeuvre des matières premières peu onéreuses. Toutefois, il est à noter que, du fait de l'utilisation d'un agent de couplage dans sa seconde étape, ce procédé mène généralement à la formation de sous-produits tels que la dicyclohexylurée. Ces sous-produits, qui ne posent pas de problèmes majeurs à l'échelle du laboratoire où une purification par chromatographie est envisageable, sont en revanche extrêmement difficiles à éliminer à l'échelle industrielle.

De ce fait, on a cherché à modifier la nature de l'étape de couplage peptidique de façon à éviter la formation de sous-produits liés à la mise en oeuvre de l'agent de couplage. Ainsi, une alternative consiste par exemple à transformer le groupement -COOH en groupement chlorure d'acide, puis à effectuer le couplage en l'absence d'agent de couplage. Cependant, si on évite effectivement la formation de sous produits de type dicyclohexylurée, l'étape de préparation du chlorure d'acide induit cependant souvent par ailleurs la formation d'autres sous-produits thiols et disulfures en quantités non négligeables, qu'il est également difficile de séparer à l'échelle industrielle.

Ainsi, il apparaît que, si elle est relativement intéressante au niveau du laboratoire, la préparation de dérivés N-(mercaptoacyl)-amino-acides à partir d'acides acryliques α-substitués, par addition de Michaël d'un thioacide et couplage peptidique d'un amino-ester, est généralement difficilement adaptable à l'échelle industrielle.

Or, les inventeurs ont maintenant découvert que, de façon surprenante, la mise en oeuvre spécifique de l'étape de couplage peptidique avant l'étape d'addition de Michaël permet d'adapter le procédé à une mise en oeuvre industrielle, en facilitant les étapes de purifications nécessaires. En effet, les travaux des inventeurs ont permis de mettre en évidence que, si l'intermédiaire chlorure d'acyle obtenu suite à une étape préalable d'addition du thioacide n'est pas purifiable à l'échelle industrielle, un chlorure d'acide obtenu directement à partir de l'acide acrylique est en revanche distillable, ce qui permet d'améliorer la pureté des dérivés N-(mercaptoacyl)-aminoacides obtenus, même dans le cadre d'un procédé industriel.

Par ailleurs, les inventeurs ont également découvert que, dans le cas où le carbone auquel est relié le groupement amino -$NH_2$ est spécifiquement un carbone asymétrique dans l'amino-ester mis en oeuvre, le couplage préalable avec l'amino-ester induit une énantiosélectivité pour la réaction ultérieure d'addition de Michaël. Cette propriété peut être avantageusement exploitée dans le cadre de la synthèse de composés de formule (I) où R2 représente un groupement alkyle inférieur, comme par exemple le fasidotril de formule (III). En effet, par mise en oeuvre d'un amino ester possédant un carbone asymétrique (S), on synthétise préférentiellement, lors de l'addition de Michaël, des composés de formule (I) sous leur forme (S,S), thérapeutiquement intéressante.

Sur la base de cette découverte, un premier but de l'invention est de fournir un procédé de préparation de

dérivés N(mercaptoacyl)-aminoacides de formule (I) exploitable au niveau industriel, et à la fois intéressant en termes de coût, de rendement et de pureté des composés obtenus.

Un second but de l'invention est de fournir un procédé de préparation énantiosélectif de composés de formule (I) où R2 est un groupement alkyle inférieur, menant préférentiellement à la formation de composés de formule (I) sous la forme (S,S) suivante :

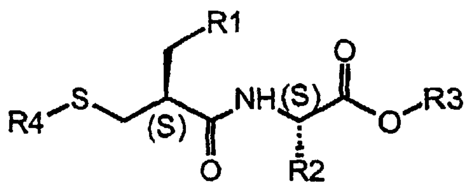

dans laquelle R1, R2, R3 et R4 ont la signification précitée,

Ainsi, la présente invention a pour objet un procédé de préparation d'un composé de formule (I) :

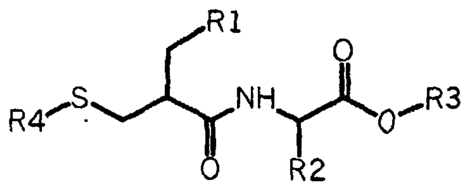

(I)

dans laquelle R1, R2, R3 et R4 ont la signification précitée, ledit procédé comprenant une étape (B) consistant à réaliser une addition de Michaël d'un thioacide de formule (IV):

R4SH (IV)

dans laquelle R4 a la définition précitée,
sur un dérivé d'amide acrylique alpha-substitué de formule (V) :

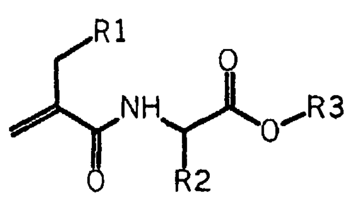

(V)

dans laquelle R1, R2 et R3 ont la même signification que dans la formule (I).

Ladite étape (B) d'addition de Michaël peut être effectuée en présence ou non d'un solvant. Le cas échéant, ce solvant est avantageusement choisi parmi le toluène, le dichlorométhane, le 1,2-dichloroéthane, l'eau, le chloroforme, le N,N-diméthylformamide, le 1,4-dioxane, le N-méthylpyrrolidone, le N,N-diméthylacétamide, l'acétate de butyle, l'acétate d'éthyle, l'acétate d'isobutyle, l'acétate d'isopropyle, l'acétate de méthyle, l'acétate de propyle et le tétrahydrofurane, le 1,4-dioxane, le cyclohexane, l'isopropanol, le n-propanol, l'acétone, le 1-butanol ou le 2-butanol.

En règle générale, l'étape (B) est par ailleurs conduite à une température comprise entre -20°C et 130°C, avantageusement entre 15°C et 115°C, et pendant une durée généralement comprise entre 1 heure et 24 heures, et de préférence comprise entre 1 heure et 6 heures.

Le composé de formule (I) préparé dans l'étape (B) peut alors être extrait du milieu réactionnel par tout moyen connu de l'homme du métier. Ainsi, dans le cadre de la mise en oeuvre d'un solvant, ledit solvant peut par exemple être partiellement évaporé, et le composé de formule (I) peut alors être obtenu par cristallisation dans un autre solvant, par exemple dans l'alcool isopropylique et/ou dans l'éther isopropylique, et par filtration et lavage.

Avantageusement, le thioacide de formule (IV) mis en oeuvre dans l'addition de Michaël de l'étape (B) est l'acide thioacétique, l'acide thiobenzoïque ou l'acide thiopivalique. En d'autres termes, le groupement R4 représente de préférence un radical acétyl $CH_3$-CO-, benzoyle $C_6H_5$-CO-, ou pivaloyle $(CH_3)_3$-CO-.

L'amide acrylique alpha-substitué de formule (V) mis en oeuvre dans l'étape (B) est quant à lui généralement obtenu à partir d'un acide acrylique alpha-substitué de formule (VI) :

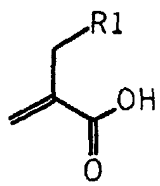

(VI)

dans laquelle R1 a la définition précitée.

En effet, ces acides acryliques de formule (VI) sont des composés peu onéreux et dont la synthèse est relativement simple à mettre en oeuvre. Pour plus de détails concernant un procédé avantageux de préparation de ces composé, on pourra notamment se reporter à la demande de brevet EP 0 729 936.

Dans ce cas, l'amide acrylique alpha-substitué de formule (V) est généralement obtenu à l'issu d'une étape (A) antérieure à l'étape (B) et comprenant une étape consistant à réaliser le couplage de l'acide acrylique de formule (VI) avec un amino-ester de formule (VIII)

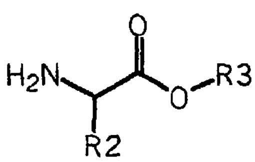

(VIII)

dans laquelle R2 et R3 ont la définition précitée.

De façon générale, l'acide acrylique (VI) est avantageusement obtenu à l'issu d'un procédé de préparation tel que décrit dans EP 0 038 758.

L'étape (A) de couplage de l'acide acrylique et de l'amino-ester peut quant à elle être conduite par tout moyen connu de l'homme du métier. Toutefois, de façon à mener à la formation d'un amide acrylique alpha-substitué (V) de pureté importante, l'étape (A) comprend avantageusement les étapes successives consistant à

(A1) faire réagir ledit acide acrylique alpha-substitué de formule (VI) avec un acide chloré, généralement inorganique, de façon à obtenir un chlorure d'acide de formule (VII) :

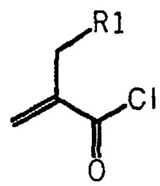

(VII)

dans laquelle R1 a la définition précitée ;

et

(A2) faire réagir le chlorure d'acide de formule (VII) ainsi obtenu avec l'amino-ester de formule (VIII), en présence d'une base, de façon à réaliser le couplage.

Généralement, l'acide chloré mis en oeuvre dans l'étape (A1) est alors choisi parmi $SOCl_2$, ClCO-COCl, $PCl_3$ ou $PCl_5$, et avantageusement parmi $SOCl_2$ ou ClCO-COCl, éventuellement en association avec le diméthylformamide. Cet acide peut être mis en oeuvre seul ou en présence d'un solvant organique, choisi de préférence, le cas échéant, parmi le toluène, un xylène, un chlorobenzène, le dichlorométhane et les mélanges de ces solvants. Avantageusement, lorsque l'acide chloré est mis en oeuvre en présence d'un solvant, ce solvant est le toluène.

Par ailleurs, quelle que soit la nature de l'acide chloré inorganique mis en oeuvre, l'étape (A1) est généralement réalisée à une température comprise entre 0°C et 130°C, de préférence entre 15°C et 120°C. Dans le cadre de la mise en oeuvre de toluène, la réaction est généralement conduite dans les conditions de reflux du toluène. La réaction de chloration de l'acide peut être réalisée par ajout progressif de l'acide chloré, le cas échéant pendant une durée généralement comprise entre 5 minutes et 7 heures-ou par addition directe de l'acide chloré. Cependant, dans tous les cas, on met généralement en oeuvre un excès d'acide inorganique, et de préférence une quantité d'acide inorganique comprise entre 1 et 2 équivalent(s) molaire(s) par rapport à l'acide acrylique, et on laisse généralement la réaction se poursuivre pendant une durée comprise entre 30 minutes et 5 heures après l'addition de l'acide halogéné.

Le chlorure d'acide de formule (VII) obtenu à l'issu de l'étape (A1) est avantageusement soumis à une étape de purification avant sa mise en oeuvre dans l'étape (A2). A ce sujet, il est à souligner que, du fait de sa structure chimique, le chlorure d'acide (VII) obtenu à l'issue de l'étape (A1) peut être soumis à une étape de distillation avant l'étape (A2) de couplage. Il est à noter qu'une telle étape de distillation est compatible avec une mise en oeuvre industrielle. De fait, le chlorure d'acide (VII) est généralement distillé avant l'étape (A2), de préférence sous une pression réduite généralement comprise entre 100 et 3000 Pa (c'est-à-dire entre 0,001 et 0,03 bars), et à une température avantageusement comprise entre 70°C et 160°C. Il est à souligner que cette possibilité de distillation, permet d'obtenir *in fine* des composés de formule (I) de pureté accrue en éliminant les sous produits éventuellement présents à l'issu de l'étape de chloration.

L'étape (A2) de formation de la liaison peptidique est spécifiquement réalisée en présence d'une base. De préférence, cette base est une base organique, et plus préférentiellement, il s'agit d'une amine organique, avantageusement choisie parmi la triéthylamine ou la diisopropyléthylamine.

Par ailleurs, il est également à noter que, notamment de façon à améliorer le rendement du couplage, l'amino-ester mis en oeuvre dans l'étape (A2) est quant à lui généralement introduit sous la forme d'un sel, et de préférence sous la forme d'un sel de formule (VIII bis) :

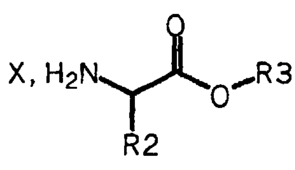

(VIII bis)

dans laquelle R2 et R3 ont les définitions précitées, et où X est choisi parmi HCl, $CH_3SO_3H$, 4-méthylphényl-$SO_3H$.

L'étape (A2) est généralement conduite en présence d'un solvant organique généralement choisi, le cas échéant, parmi le toluène, le dichlorométhane, le 1,2-dichloroéthane, le chloroforme, le N,N-diméthylformamide, le 1,4-dioxane, la N-méthylpyrrolidone, le N,N-diméthylacétamide, l'acétate de butyle, l'acétate d'éthyle, l'acétate d'isobutyle, l'acétate d'isopropyle, l'acétate de méthyle, l'acétate de propyle ou encore le tétrahydrofurane.

Il est à souligner que la réaction d'addition du chlorure d'acide (VII) sur l'amino-ester (VIII) ou son sel (VIIIbis) réalisée dans l'étape (A2) est généralement fortement exothermique. De ce fait, l'étape (A2) est généralement conduite à une température faible, avantageusement comprise entre -10°C et 25°C, et la réaction est de préférence conduite par addition progressive du chlorure d'acide (VII) dans un milieu maintenu à la température de réaction souhaitée et contenant l'amino-ester ou le sel de amino-ester, et la base. En règle générale, la base est mise en oeuvre en une quantité supérieure à deux équivalents molaires par rapport à la quantité de chlorure d'acide introduite. L'amino-ester (VIII) est quant à lui avantageusement mis en oeuvre en léger excès par rapport au chlorure d'acide (VII), et de préférence à raison d'une quantité comprise entre 1 et 1,2 équivalent molaire par rapport au chlorure d'acide. De préférence, le chlorure d'acide est additionné en une durée comprise entre 30 mn et 3 heures, et on laisse généralement la réaction d'addition se poursuivre à la température de travail choisie pendant une durée avantageusement comprise entre 15 minutes et 3 heures.

Une fois l'addition réalisée, le produit (VI) issu de la réaction est généralement isolé par extraction, le cas échéant par lavage-du milieu réactionnel par de l'eau et/ou par une solution aqueuse acide, suivie d'une séparation de la phase organique. Avantageusement la phase organique est alors soumise à un ou plusieurs lavages ultérieurs par de l'eau et/ou par des solutions aqueuses de préférence acides. Le solvant présent est alors généralement éliminé, par exemple par évaporation et/ou par cristallisation du composé (VI), filtration et séchage.

Selon un mode de réalisation particulièrement avantageux du procédé de l'invention, le composé (V) mis en oeuvre dans l'étape (B) est un composé chiral où R2 désigne spécifiquement un groupement alkyle inférieur, obtenu par exemple par couplage d'un amino-ester chiral de formule (VIII) présentant le même groupement R2 et d'un acide acrylique de formule (VI).

Dans ce cas, en effet, dans la mesure où R2 n'est pas un atome d'hydrogène, l'atome de carbone auquel est rattaché le groupement R2 est spécifiquement un carbone asymétrique. De ce fait, le composé (V) est un composé chiral. L'orientation du groupement R2 au sein de ce composé induit donc une stéréosélectivité pour la réaction de Michaël de l'étape (B) ultérieure. De ce fait, on préfère mettre en oeuvre le composé (V) au moins majoritairement sous sa configuration S ou au moins majoritairement sous sa configuration R.

De façon particulièrement avantageuse, dans le composé (V), le carbone asymétrique lié au groupement R2 est un carbone de configuration S. En effet, dans ce cas, le composé de formule (I) obtenu de façon majoritaire à l'issu de l'étape (B) est un composé (I) de configuration (S,S) qui présente une activité thérapeutique intéressante, notamment une activité inhibitrice sur certaines enzymes, telles l'endopeptidase neutre et l'enzyme de conversion de l'angiotensine. Ainsi, dans le cadre de la préparation du fasidotril de formule (III), la mise en oeuvre du (S)-alaninate de benzyle à titre d'amino-ester de formule (VIII) mène à la formation préférentielle du composé (I) correspondant, majoritairement sous la forme (S,S) de la formule (III), alors que la forme (R,S), qui ne présente pas d'intérêt au niveau thérapeutique, est obtenue en quantité minoritaire.

La mise en oeuvre d'un composé (V) de configuration (R) mène de la même façon à l'obtention majoritaire d'un composé (I) de configuration (R,R). Toutefois, de tels composés (R,R) ne présentent généralement pas d'activité thérapeutique intéressante.

De ce fait, on préfère mettre en oeuvre le composé (V) sous sa forme S optiquement pure, de façon à favoriser la formation de composés (I) de configuration(S,S). Pour ce faire, le composé (V) est généralement préparé par une réaction de condensation d'un acide acrylique de formule (VI) avec un amino-ester de formule (VIII) dérivé d'un acide aminé naturel tel que l'alanine, qui présente naturellement un groupement alkyle inférieur sur un carbone de configuration S.

De façon à quantifier la stéréosélectivité de la réaction de Michaël de l'étape (B), il est à noter qu'on peut définir, de façon générale, pour les composés de formule (I) obtenus à l'issu de l'étape (B), un excès énantiomérique S : R exprimé par le rapport molaire :

$$(n_S-n_R) / (n_S+n_R),$$

où :

- $n_S$ représente le nombre de mole de composé (I) où le carbone portant le groupement $-CH_2-R1$ est de configuration S ;
  et

- $n_R$ représente le nombre de mole de composé (I) où le carbone portant le groupement $-CH_2$-R1 est de configuration R.

En règle générale, lorsque R2 est un atome d'hydrogène, cet excès énantiomérique S : R est nul, et les deux énantiomères R et S ont autant de chance de se former lors de l'addition de Michaël de l'étape B. Ainsi, par exemple, le racécadotril de formule (II), obtenu à partir d'un composé (V) où R2=H, est synthétisé sous la forme d'un mélange racémique par le procédé de l'invention.
En revanche, dans le cas où R2 n'est pas un atome d'hydrogène, la chiralité du composé (V) induit une énantioselectivité pour la réaction de Michaël. De ce fait, lors de la mise en oeuvre spécifique d'un intermédiaire réactionnel de type (V) chiral au moins majoritairement sous sa configuration S ou au moins majoritairement sous sa cofiguration R, l'excès énantiomérique S : S observé pour les composés (I) issus de l'étape (B), n'est pas nul.
Ainsi, dans le cadre de la mise en oeuvre d'un dérivé d'amide acrylique optiquement pur où le carbone asymétrique lié au groupement R2 est un carbone de configuration S, obtenu par exemple par couplage d'un amino-ester (VIII) de configuration (S) avec un acide acrylique (VI), l'excès énantiomérique S : R défini précédemment , qui correspond alors à un excès diastéréoisomérique (S,S) : (R,S), est généralement supérieur à 10 %, et il peut même être dans certains cas supérieur à 25 %, voire supérieur à 30 %.
Toutefois, dans le cadre de l'utilisation de composés (V) chiraux, de façon à améliorer encore la stéréosélectivité de la réaction, on peut, dans certains cas, mettre en oeuvre dans la réaction de Michaël de l'étape (B) des inducteurs de chiralité, tels que par exemple des alkaloïdes du quinquina tels que, par exemple, la quinine, la quinidine, la cinchonine ou la cinchonidine, ou des dérivés de ces composés, comme par exemple la o-acétylquinine, et, le cas échéant, de préférence à raison de 0,01 à 1 équivalent par rapport au composé (V). Dans le cas général, la présence de tels composés n'est toutefois absolument pas nécessaire pour obtenir un excès énantiomérique supérieur à 15 %.
Par ailleurs, dans le cas spécifique où R2 est un groupement alkyle inférieur conférant au carbone sur lequel il est fixé un caractère chiral, le procédé de l'invention peut également comprendre, à l'issue de l'étape (B) une étape ultérieure (C) de séparation des diastéréoisomères obtenus dans l'étape (B).
Ainsi, dans le cadre de la mise en oeuvre d'un composé (V) de configuration (S) préférentiellement utilisé, une étape de séparation des diastéréoisomères de configurations (S,S) et (R,S) obtenus à l'issu de l'étape (B) peut généralement être réalisé par cristallisation sélective du composé (S,S), le cas échéant dans un solvant ou dans un mélange de solvants choisi(s) avantageusement parmi l'isopropanol, le n-propanol, l'éthanol, le méthanol, l'éther diisopropylique, le toluène, le dichlorométhane, le chloroforme, le 1,4-dioxane, l'acétate de butyle, l'acétate d'éthyle, l'acétate d'isobutyle, l'acétate d'isopropyle, l'acétate de méthyle, l'acétate de propyle, le tétrahydrofurane, le 1,4-dioxane, le cyclohexane, l'acétone, le 1-butanol , le 2-butanol, le cyclohexane ou le 1,2-diméthoxyéthane.
Les caractéristiques et avantages du procédé de l'invention apparaîtront encore plus nettement au vu des exemples non limitatifs exposés ci-après.

<u>Exemple 1: préparation du racécadotril de formule (II) (N-(RS)-[2-acétylthiométhyl-1-oxo-3-phényl-propyl]-glycinate de benzyle).</u>

Etape (A) : Préparation du N-[1-oxo-2-benzyl-propènyl]-glycinate de benzyle.

<u>Etape (A1) : synthèse du chlorure de l'acide 2-benzyl propènoïque</u>

Dans un erlenmeyer de 500 ml, on a introduit :

- 71,49 g (soit 441,29 mmoles) d'acide benzylacrylique ; et
- 179 ml de toluène.

On a chauffé à une température de 110° C le mélange obtenu et on a distillé environ 35 ml de toluène, de façon à sécher l'acide benzylacrilyque par entraînement azéotropique.
On a ensuite laissé la température se stabiliser à 70°C, puis on a ajouté, en une durée de 5 heures, 64,06 g (soit 538,32 mmoles) de chlorure de thionyle $SOCl_2$. La température a été maintenue égale à 70°C pendant toute la durée de l'addition.
Suite à l'addition, le mélange réactionnel a été maintenu 3 heures à 70°C.
On a ensuite laissé la température diminuer jusqu'à la température ambiante (25°C), puis on a concentré le milieu réactionnel par évaporation du toluène sous évaporateur rotatif sous vide.
On a alors obtenu 82,8 g d'huile jaune-orangée.
On a ensuite distillé l'huile sous le vide obtenu à l'aide d'une trompe à eau (15 mmHg), en chauffant à l'aide

d'un bain d'huile à une température comprise entre 145 et 150°C.

On a alors obtenu 72,71 g d'une huile incolore.

Point d'ébullition : 115°C

Rendement de l'étape (A1): 91%

Etape (A2) : couplage avec le paratoluènesulfonate de glycinate de benzyle

Dans un erlenmeyer de 250 ml, on a introduit :

- 45,18 g (soit 134,06 mmol) de paratoluènesulfonate de glycinate de benzyle ; et
- 110 ml de toluène.

La suspension a été agitée et refroidie par immersion dans un bain de glace à 0°C.

On a ajouté en 30 minutes, en maintenant la température à 0°C, 27,08 g (soit 268,11 millimoles) de triéthylamine.

Une fois le milieu devenu limpide, on a maintenu la température à 0°C pendant 30 minutes.

On a ensuite ajouté, sous agitation et de façon progressive (en une heure) 22 g (soit 121,88 millimoles) du chlorure d'acide obtenu sous forme d'huile incolore dans l'étape (A1) précédente, en maintenant toujours l'erlenmeyer dans un bain de glace à 0°C, du fait de l'exothermie de la réaction.

On a laissé le mélange réactionnel sous agitation pendant une heure à 0°C, puis on a laissé la température remonter jusqu'à 25°C.

On a alors ajouté 45 ml d'eau distillée dans le milieu, puis on a acidifié à un pH de 4 par ajout d'acide chlorhydrique 5N. On a agité pendant 5 minutes puis on a versé les deux phases obtenues dans une ampoule à décanter. La phase organique a été séparée puis successivement lavée avec :

- 1 ) 90 ml d'eau
- 2) 45 ml d'une solution aqueuse de bicarbonate de sodium à 44 g par litre
- 3) 45 ml d'eau

La phase organique a ensuite été concentrée sous évaporateur rotatif sous vide de façon à éliminer le solvant.

Le mélange concentré obtenu a été dissous dans 110 ml d'un mélange d'éther isopropylique et d'alcool isopropylique 75/25 en volume. On a agité puis on a refroidit la solution obtenue. Une cristallisation a commencé à 8°C et s'est avéré ensuite très rapide. On a laissé sous agitation pendant 30 minutes à 5°C.

On a filtré, essoré et rincé avec 20 ml d'éther isopropylique à 5°C, de façon à obtenir 30,51 g d'un solide humide que l'on a séché sous vide (20 mmHg) jusqu'à l'obtention d'une masse constante.

On a obtenu 30,0 g d'un solide blanc

Point de fusion = 52-53°C

CCM : éluant 50/50 éther éthylique/éther de pétrole, une seule tache ; Rf = 0,5

Rendement de l'étape (A2) = 79%

Etape (B) : Addition de Michaël de l'acide thioacétique.

Dans un erlemeyer de 250 ml muni d'une agitation magnétique, on a introduit 30 g (soit 97,08 mmol) du solide blanc obtenu à l'issu de l'étape (A2).

On a alors agité et on a ajouté progressivement, à 25°C, et en une durée de 30 minutes, 8,85 g (116,44 mmol) d'acide thioacétique.

On a ensuite élevé la température du milieu réactionnel jusqu'à 80°C et on a maintenu cette température pendant 3 heures.

On a ensuite fait diminuer la température jusqu'à 40°C et on a alors ajouté 19 ml d'alcool isopropylique. On a ensuite évaporé sous vide à l'évaporateur rotatif. Le résidu huileux obtenu a été dissout dans 150 ml d'alcool isopropylique et porté à 40°C. On a agité et laissé refroidir lentement. On a observé l'apparition des premiers cristaux à la température de 27°C. On a alors maintenu la température à 27°C pendant 45 minutes puis on a refroidi jusqu'à 10°C.

On filtré le solide cristallisé obtenu sur un fritté de porosité N°2, on l'a essoré, puis rincé avec 50 ml d'un mélange d'éther isopropylique et d'alcool isopropylique (3/2 en volume) à 5°C.

On a alors obtenu un solide blanc que l'on a séché à 45°C sous vide (15 mmHg).

Masse obtenue : 28,22 g

Point de fusion = 79-80°C (microscope)

CCM : éluant 50/50 éther éthylique/éther de pétrole, une seule tache ; Rf = 0,45

Rendement de l'étape (B) = 75%
Rendement global du procédé de préparation du racécadotril: 53,9%

Exemple 2 : préparation du fasidotril de formule (III) (N-(S)-[2-acétylthiométhyl-1-oxo-3-(3,4-méthylènedioxy phényl)-propyl]-(S)-alaninate de benzyle)

Etape (A) : Préparation du N-[1-oxo-2-(3,4-méthylènedioxy-benzyl)-propènyl]-(S)-alaninate de benzyle.

Etape (A1) : synthèse du chlorure de l'acide 2-(3,4 méthylènedioxy benzyl)-propénoïque.:

Dans un tricol de 250 ml, on a introduit :

- 50 g(soit 242,71 millimoles) d'acide pipéronylacrylique ; et
- 50 ml de toluène

On a ajouté, en une durée de 5 minutes, 34,66 g (soit 291,26 millimoles) de chlorure de thionyle.
On a chauffé à reflux la suspension réalisée à l'aide d'un bain d'huile pendant 30 minutes.
On a ensuite laissé le milieu réactionnel revenir à température ambiante (25°C) puis on l'a concentré à l'évaporateur rotatif dans un bain d'eau à 45°C.
On a alors obtenu une huile brute de couleur rouge-orangée.
Masse = 56,95 g

Etape (A2) : couplage avec le méthanesulfonate d'alaninate de benzyle

Dans un tricol d'un litre, on a introduit :

- 150 ml de toluène ; et
- 66,75 g (soit 242,72 mmol) de méthanesulfonate d'alaninate de benzyle.

On a agité la suspension obtenue et on l'a refroidie à 5°C.
On a lors ajouté en 15 minutes 51,48 g (509,70 mmol) de triéthylamine.
On a agité 10 minutes à une température située entre 0 et +5°C.
On a ensuite coulé lentement dans le milieu les 56,95 g d'huile brute obtenue à l'issu de l'étape (A1) en solution dans 50 ml de toluène sans dépasser 15°C dans la masse lors de l'addition. Compte tenu de la très forte exothermie de la réaction, l'addition a été effectuée en 40 minutes en maintenant le tricol dans un bain de glace additionné de sel, à -5°C.
On a ensuite laissé la température du milieu réactionnel s'élever jusqu'à la température ambiante (25°C) en environ 20 minutes. On a alors ajouté 150 ml d'eau et on a agité pendant 5 minutes.
On a transféré les deux phases obtenues dans une ampoule à décanter et on a éliminé la phase aqueuse.
On a ensuite lavé la phase toluénique successivement par :

- 100 ml d'HCl 1N
- 150 ml d'eau

La phase organique a alors été concentrée sous évaporateur rotatif dans un bain d'eau à 50°C.
On a obtenu 87,16 g de résidu huileux que l'on a dissous dans 27 ml d'isopropanol.
On a ensuite ajouté 100 ml d'éther isopropylique.
La solution obtenue a été transférée dans un bécher puis refroidie à 10°C et agitée.
On a alors ensemencé le milieu alors avec 5 mg de N-[1-oxo-2-(3,4-méthylènedioxy-benzyl)-propènyl]-(S)-alaninate de benzyle sous forme cristallisée.
On a ensuite refroidi le milieu jusqu'à 5°C et on a agité pendant 30 minutés.
On a filtré sur un fritté de porosité N°2 le solide cristallisé formé.
Le précipité a ensuite été reempâté avec 100 ml d'éther isopropylique à 5°C puis filtré, puis on l'a rincé avec 25 ml d'éther isopropylique à 5°C, et séchée sous vide jusqu'à masse constante.
On obtient 63,93 g d'un solide blanc-crème.
Point de fusion : 51-52°C (au microscope)
CCM : éluant 50/50 éther éthylique/éther de pétrole ; révélateur : acide phosphomolybdique ; Rf = 0,38.
Pouvoir rotatoire : $[\alpha]_D$ = -16,7° (25°C, c = 2,04 dans le méthanol).

Rendement de l'étape (A) : 71%

Etape (B) : Addition de Michaël de l'acide thioacétique.

Dans un tricol de 250 ml muni d'une agitation magnétique, on a introduit 30 g (81,74 mmol) du solide obtenu à l'issu de l'étape précédente.

On a alors agité et on a introduit progressivement, à 25°C, et en une durée de 5 minutes, 7,76 g (soit 102,10 mmol) d'acide thioacétique.

On a ensuite chauffé le milieu réactionnel pendant 2 à 3 heures à 80°C.

Le milieu huileux alors obtenu a été soumis à une analyse par RMN $^1H$ et HPLC, de façon à contrôler la fin de la réaction. L'excès diastéréoisomérique (S,S) : (R,S) au sein du milieu réactionnel a alors été mesuré égal à 25 %.

Etape (C1) : Première recristallisation.

On a ensuite ajouté au milieu réactionnel issu de l'étape (B) 300 ml d'isopropanol, et on a refroidi la solution obtenue à 30°C.

On a ensuite ensemencé le milieu avec 50 mg de fasidotril (S,S) cristallisé et on a maintenu la température à 30°C pendant 2 heures.

On a filtré et rincé le solide obtenu avec 15 ml d'isopropanol, puis on l'a séché sous vide (20 mmHg) à 25°C.

On a alors obtenu 13,88 d'un solide blanc

Point de fusion : 97-100°C

CCM : éluant : éther éthylique/éther de pétrole 60/40 ; Rf = 0,44, une seule tache ; révélateur: UV 254 nm et acide phosphomolybdique.

RMN $^1H$ : la RMN $^1H$ met en évidence un excès diastéréoisomérique (S,S) : (S,R) d'environ 90%.

Rendement global des étapes (B) et (C1) : 38%

Etape (C2) : seconde recristallisation.

Dans un ballon tricol de 250 ml, on a introduit les 13,88 g de solide blanc obtenu précédemment ainsi que 207 ml d'isopropanol.

On a agité et chauffé à une température de 70°C jusqu'à dissolution complète du solide.

On a alors refroidi la solution obtenue à 50°C et on a amorcé la cristallisation sélective du composé (S,S) par introduction de quelques cristaux de fasidotril (S,S).

On a alors refroidi le milieu jusqu'à 35°C en 2 heures et on a ensuite maintenu la température à 35°C pendant 1 heure 30.

On a ensuite filtré sur un fritté de porosité N°2 le solide cristallisé obtenu et on l'a rincé avec 15 ml d'isopropanol.

Après séchage sous vide (20 mmHg) à 25°C, on obtient ainsi 11,16 g d'un solide.

Point de fusion : 109°C (microscope)

Excès diastéréoisomérique (S,S) : (R,S) : au moins égal à 98 %.

CCM : éluant 60/40 éther éthylique/éther de pétrole ; révélateur : UV 254 nm et acide phosphomolybdique ; Rf = 0,42.

Pouvoir rotatoire : $[\alpha]_D$ = -51,8° (20°C, c = 1,03 dans le méthanol).

Rendement de l'étape (C) : 80%

Rendement global du procédé de préparation du fasidotril : 30%

## Revendications

1. Procédé de préparation d'un composé de formule (I) :

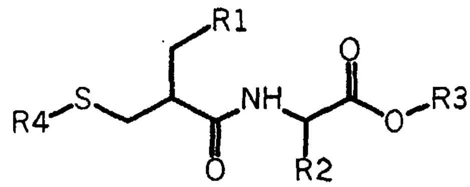

(I)

dans laquelle :

- R1 représente :
  - un groupe phényle ; ou
  - un groupe 3,4-méthylènedioxy-phényle ;
- R2 représente un atome d'hydrogène ou un groupe alkyle en $C_1$-$C_6$ ;
- R3 représente un atome d'hydrogène, un groupe alkyle en $C_1$-$C_6$ ou un groupe phénylalkylène en $C_1$-$C_6$ ; et
- R4 représente un radical acyle aliphatique linéaire ou ramifié ou un radical acyle aromatique,

ledit procédé comprenant une étape (B) consistant à réaliser une addition de Michaël d'un thioacide de formule (IV):

R4SH (IV)

dans laquelle R4 a la même signification que dans la formule (I), sur un dérivé d'amide acrylique alpha-substitué de formule (V) :

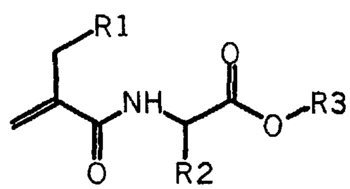

(V)

dans laquelle R1, R2 et R3 ont la même signification que dans la formule (I).

2. Procédé selon la revendication 1, **caractérisé en ce que** le groupement R4 représente un radical acétyl $CH_3$-CO-, benzoyle $C_6H_5$-CO-, ou pivaloyle $(CH_3)_3$-CO-.

3. Procédé la revendication 1 ou la revendication 2, **caractérisé en ce que** ledit dérivé d'amide acrylique alpha-substitué de formule (V) est obtenu à l'issu d'une étape (A) antérieure à l'étape (B) et comprenant une étape consistant à réaliser le couplage d'un acide acrylique de formule (VI) :

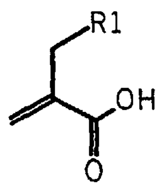

(VI)

dans laquelle R1 a la même signification que dans la formule (I), avec un amino-ester de formule (VIII) :

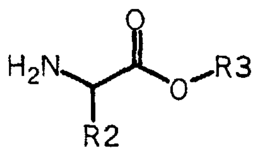

(VIII)

dans laquelle R2 et R3 ont la même signification que dans la formule (I).

**4.** Procédé selon la revendication 3, **caractérisé en ce que** ladite étape (A) comprend les étapes successives consistant à :

(A1) faire réagir ledit acide acrylique alpha-substitué de formule (VI) avec un acide inorganique chloré, de façon à obtenir un chlorure d'acide de formule (VII) :

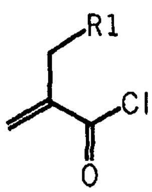

(VII)

dans laquelle R1 a la même signification que dans la formule (I); et

(A2) faire réagir le chlorure d'acide de formule (VII) ainsi obtenu avec ledit amino-ester de formule (VIII), en présence d'une base, de façon à réaliser le couplage.

**5.** Procédé selon la revendications 4, **caractérisé en ce que** l'acide inorganique chloré mis en oeuvre dans l'étape (A1) est choisi parmi $SOCl_2$, ClCO-COCl, $PCl_3$ ou $PCl_5$.

**6.** Procédé selon la revendication 4 ou la revendication 5, **caractérisé en ce que** le chlorure d'acide de formule (VII) obtenu à l'issu de l'étape (A1) est soumis à une étape de distillation avant sa mise en oeuvre dans l'étape (A2).

**7.** Procédé selon l'une quelconque des revendications 4 à 6, **caractérisé en ce que** la base mise en oeuvre dans l'étape (A2) est une amine organique.

**8.** Procédé selon l'une quelconque des revendications 4 à 7, **caractérisé en ce que** l'amino-ester mis en oeuvre dans l'étape (A2) est introduit sous la forme d'un sel de formule (VIII bis) :

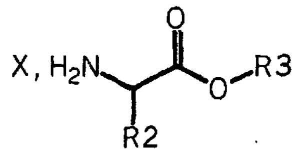

(VIII bis)

dans laquelle R2 et R3 ont la même signification que dans la formule (I) ; et où X est choisi parmi HCl, $CH_3SO_3H$, et 4-méthylphényl-$SO_3H$.

9. Procédé selon l'une quelconque des revendications 4 à 8, **caractérisé en ce que** l'étape (A2) est conduite en présence d'un solvant organique choisi parmi le toluène, le dichlorométhane, le 1,2-dichloroéthane, le chloroforme, le N,N-diméthylformamide, le 1,4-dioxane, la N-méthylpyrrolidone, le N,N-diméthylacétamide, l'acétate de butyle, l'acétate d'éthyle, l'acétate d'isobutyle, l'acétate d'isopropyle, l'acétate de méthyle, l'acétate de propyle ou le tétrahydrofurane.

10. Procédé selon l'une quelconque des revendications 1 à 9, **caractérisé en ce que** le composé (V) mis en oeuvre dans l'étape (B) est un composé chiral où R2 désigne un groupement alkyle inférieur, ledit composé (V) étant mis en oeuvre au moins majoritairement sous sa configuration S ou au moins majoritairement sous sa configuration R.

11. Procédé selon la revendication 10, **caractérisé en ce que**, le composé (V) est mis en oeuvre sous sa forme S optiquement pure.

12. Procédé selon la revendication 11, **caractérisé en ce que** le composé (V) est préparé par une réaction de condensation d'un acide acrylique de formule (VI) avec un amino-ester de formule (VIII) dérivé d'un acide aminé naturel.

13. Procédé selon l'une quelconque des revendications 10 à 12, **caractérisé en ce qu'**on met en oeuvre dans l'étape (B) des inducteurs de chiralité.

14. Procédé selon l'une quelconque des revendications 10 à 12, **caractérisé en ce qu'**il comprend, à l'issue de l'étape (B), une étape ultérieure (C) de séparation des diastéréoisomères obtenus dans l'étape (B).

15. Procédé selon l'une quelconque des revendications 1 à 9, **caractérisé en ce que** ledit composé de formule (I) obtenu est le N-(RS)-[2-acétylthiométhyl-1-oxo-3-phényl-propyl]-glycinate de benzyle de formule (II) :

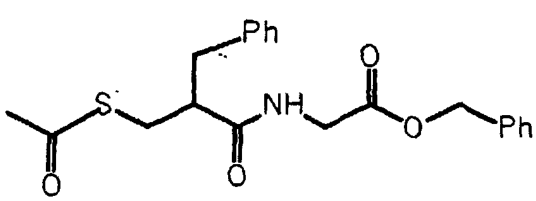

(II)

16. Procédé selon l'une quelconque des revendications 1 à 14, **caractérisé en ce que** ledit composé de formule (I) obtenu est le N-(S)-[2-acétylthiométhyl-1-oxo-3-(3,4-méthylènedioxy-phényl)-propyl]-(S)-alaninate de benzyle de formule (II) :

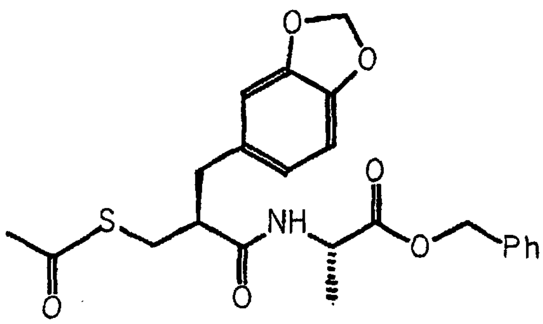

(III)

## Patentansprüche

**1.** Verfahren zur Herstellung einer Verbindung mit der Formel (I)

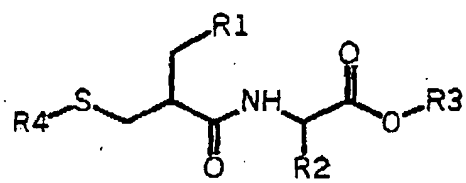

(I),

in welcher

- $R_1$ eine Phenylgruppe oder eine 3,4-Methylendioxyphenylgruppe,
- $R_2$ ein Wasserstoffatom oder eine $C_1$- bis $C_6$-Alkylgruppe,
- $R_3$ ein Wasserstoffatom, eine $C_1$- bis $C_6$-Alkylgruppe oder eine $C_1$- bis $C_6$-Phenylalkylengruppe und
- $R_4$ einen geradkettigen oder verzweigten aliphatischen $C_1$- bis $C_6$-Acylrest oder einen aromatischen Acylrest bedeutet,

wobei das Verfahren eine Stufe (B) umfasst, die in einer Michael-Addition einer Thiosäure mit der Formel (IV)

$$R_4SH \quad (IV),$$

in welcher $R_4$ dieselbe Bedeutung wie in Formel (I) hat, an ein $\alpha$-substituiertes Acrylamidderivat mit der Formel (V)

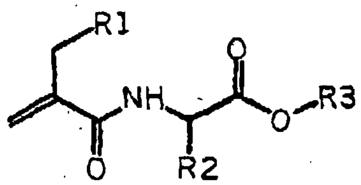

(V),

in welcher $R_1$, $R_2$ und $R_3$ dieselbe Bedeutung wie in Formel (I) haben, besteht.

2. Verfahren nach Anspruch 1, **dadurch gekennzeichnet, dass** der Rest $R_4$ einen Acetyl-, $CH_3$-CO-, Benzoyl-, $C_6H_5$-CO-, oder Pivaloylrest, $(CH_3)_3$-CO-, bedeutet.

3. Verfahren nach Anspruch 1 oder 2, **dadurch gekennzeichnet, dass** das α-substituierte Acrylamidderivat mit der Formel (V) nach einer Stufe (B) vorhergehenden Stufe (A) erhalten worden ist, und welches eine Stufe umfasst, die in der Anlagerung einer Acrylsäure mit der Formel (VI)

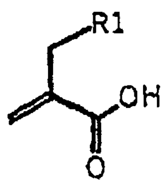

(VI),

in welcher $R_1$ dieselbe Bedeutung wie in Formel (I) hat, an einen Aminosäureester mit der Formel (VIII)

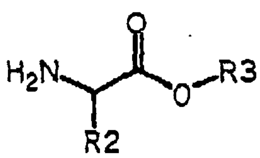

(VIII),

in welcher $R_2$ und $R_3$ dieselbe Bedeutung wie in Formel (I) haben, besteht.

4. Verfahren nach Anspruch 3, **dadurch gekennzeichnet, dass** die Stufe (A) die aufeinanderfolgenden Stufen umfasst, die im

- ($A_1$) derartigen Umsetzen der α-substituierten Acrylsäure mit der Formel (VI) mit einer chlorierten anorganischen Säure, dass ein Säurechlorid mit der Formel (VII)

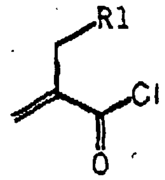

(VII),

in welcher $R_1$ dieselbe Bedeutung wie in Formel (I) hat, erhalten wird, und

- ($A_2$) derartigen Umsetzen des so erhaltenen Säurechlorids mit der Formel (VII) mit dem Aminosäureester mit der Formel (VIII) in Gegenwart einer Base, dass die Anlagerung erfolgt,

bestehen.

**5.** Verfahren nach Anspruch 4, **dadurch gekennzeichnet, dass** die in Stufe ($A_1$) eingesetzte chlorierte anorganische Säure aus $SOCl_2$, ClCO-COCl, $PCl_3$ oder $PCl_5$ ausgewählt ist.

**6.** Verfahren nach Anspruch 4 oder 5, **dadurch gekennzeichnet, dass** das nach Stufe ($A_1$) erhaltene Säurechlorid mit der Formel (VII) vor seiner Verwendung in der Stufe ($A_2$) einer Destillationsstufe unterworfen wird.

**7.** Verfahren nach einem der Ansprüche 4 bis 6, **dadurch gekennzeichnet, dass** die in Stufe ($A_2$) eingesetzte Base ein organisches Amin ist.

**8.** Verfahren nach einem der Ansprüche 4 bis 7, **dadurch gekennzeichnet, dass** der in Stufe ($A_2$) eingesetzte Aminosäureester in Form eines Salzes mit der Formel (VIIIa)

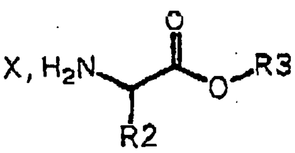

(VIIIa),

in welcher $R_2$ und $R_3$ dieselbe Bedeutung wie in Formel (I) haben und X aus HCl, $CH_3SO_3H$ und 4-Methylphenyl-$SO_3H$ ausgewählt wird,
zugegeben wird.

**9.** Verfahren nach einem der Ansprüche 4 bis 8, **dadurch gekennzeichnet, dass** die Stufe ($A_2$) in Gegenwart eines organischen Lösungsmittels durchgeführt wird, das aus Toluol, Dichlormethan, 1,2-Dichlorethan, Chloroform, N, N-Dimethylformamid, 1,4-Dioxan, N-Methylpyrrolidon, N,N-Dimethylacetamid, Butylacetat, Ethylacetat, Isobutylacetat, Isopropylacetat, Methylacetat, Propylacetat oder Tetrahydrofuran ausgewählt ist.

**10.** Verfahren nach einem der Ansprüche 1 bis 9, **dadurch gekennzeichnet, dass** die in Stufe (B) eingesetzte Verbindung (V) eine chirale Verbindung ist, worin $R_2$ eine niedere Alkylgruppe bedeutet, wobei die Verbindung (V) wenigstens überwiegend in S-Konfiguration oder wenigstens überwiegend in R-Konfiguration eingesetzt wird.

**11.** Verfahren nach Anspruch 10, **dadurch gekennzeichnet, dass** die Verbindung (V) in optisch reiner S-Form eingesetzt wird.

**12.** Verfahren nach Anspruch 11, **dadurch gekennzeichnet, dass** die Verbindung (V) durch eine Kondensationsreaktion einer Acrylsäure mit der Formel (VI) mit einem von einer natürlichen Aminosäure abgeleiteten Aminosäureester mit der Formel (VIII) hergestellt wird.

**13.** Verfahren nach einem der Ansprüche 10 bis 12, **dadurch gekennzeichnet, dass** in Stufe (B) Induktoren für die Chiralität eingesetzt werden.

**14.** Verfahren nach einem der Ansprüche 10 bis 12, **dadurch gekennzeichnet, dass** es nach Stufe (B) eine anschließende Trennungsstufe (C) für die in Stufe (B) erhaltenen Diastereoisomeren umfasst.

**15.** Verfahren nach einem der Ansprüche 1 bis 9, **dadurch gekennzeichnet, dass** die erhaltene Verbindung mit der Formel (I) N-(RS)-[2-Acetylthiomethyl-1-oxo-3-phenylpropyl]-benzylglycinat mit der Formel (II) ist:

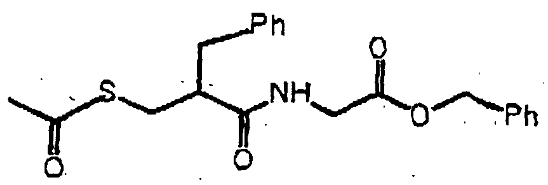

(II).

**16.** Verfahren nach einem der Ansprüche 1 bis 14, **dadurch gekennzeichnet, dass** die erhaltene Verbindung mit der Formel (I) N-(S)-[2-Acetylthiomethyl-1-oxo-3-(3,4-methylendioxyphenyl)-propyl]-(S)-benzylalaninat mit der Formel (III) ist:

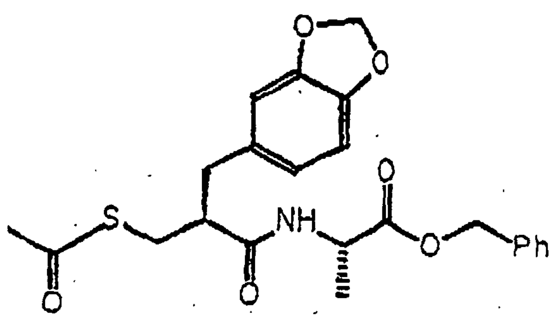

(III).

## Claims

**1.** Process for the preparation of a compound of formula (I) :

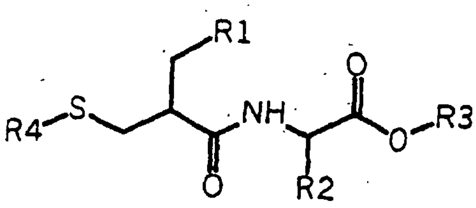

(I)

in which:

- R1 represents:
  - a phenyl group; or
  - a 3,4-methylenedioxy-phenyl group;

- R2 represents a hydrogen atom or a $C_1$-$C_6$alkyl group;
- R3 represents a hydrogen atom, a $C_1$-$C_6$alkyl group or a $C_1$-$C_6$phenylalkylene group; and
- R4 represents a linear or branched aliphatic acyl radical or an aromatic acyl radical,

said process comprising a step (B) which consists in . carrying out the Michael addition of a thioacid of formula (IV) :

R4SH (IV)

in which R4 is as defined for formula (I),
with an alpha-substituted acrylic amide derivative of formula (V):

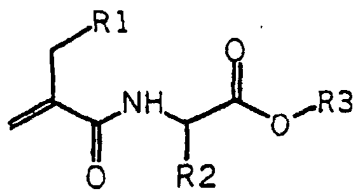

(V)

in which R1, R2 and R3 are as defined for formula (I).

2. Process according to claim 1, **characterised in that** the grouping R4 represents an acetyl radical $CH_3$-CO-, a benzoyl radical $C_6H_5$-CO- or a pivaloyl radical $(CH_3)_3$-CO-.

3. Process according to either claim 1 or claim 2, **characterised in that** said alpha-substituted acrylic amide derivative of formula (V) is obtained following a step (A) which precedes step (B) and comprises a step which consists in effecting the coupling of an acrylic acid of formula (VI) :

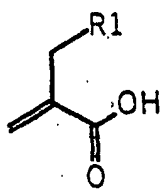

(VI)

in which R1 is as defined for formula (I),
with an amino ester of formula (VIII) :

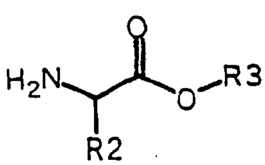

(VIII)

in which R2 and R3 are as defined for formula (I).

4. Process according to claim 3, **characterised in that** said step (A) comprises successive steps which consist in:

(A1) reacting said alpha-substituted acrylic acid of formula (VI) with a chlorinated inorganic acid in order to obtain an acid chloride of formula (VII) :

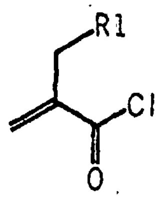

(VII)

in which R1 is as defined for formula (I) ;
and

(A2) reacting the acid chloride of formula (VII) so obtained with said amino ester of formula (VIII), in the presence of a base, in order to effect the coupling.

**5.** Process according to claim 4, **characterised in that** the chlorinated inorganic acid used in step (A1) is selected from $SOCl_2$, ClCO-COCl, $PCl_3$ and $PCl_5$.

**6.** Process according to either claim 4 or claim 5, **characterised in that** the acid chloride of formula (VII) obtained following step (A1) is subjected to a distillation step before being used in step (A2).

**7.** Process according to any one of claims 4 to 6, **characterised in that** the base used in step (A2) is an organic amine.

**8.** Process according to any one of claims 4 to 7, **characterised in that** the amino ester used in step (A2) is introduced in the form of a salt of formula (VIIIa) :

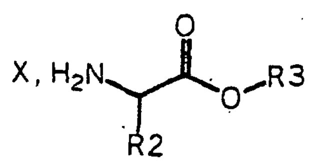

(VIIIa)

in which R2 and R3 are as defined for formula (I) ; and
wherein X is selected from HCl, $CH_3SO_3H$ and 4-methyl-phenyl-$SO_3H$.

**9.** Process according to any one of claims 4 to 8, **characterised in that** step (A2) is carried out in the presence of an organic solvent selected from toluene, dichloromethane, 1,2-dichloroethane, chloroform, N,N-dimethyl-formamide, 1,4-dioxane, N-methylpyrrolidone, N,N-dimethylacetamide, butyl acetate, ethyl acetate, isobutyl acetate, isopropyl acetate, methyl acetate, propyl acetate and tetrahydrofuran.

**10.** Process according to any one of claims 1 to 9, **characterised in that** the compound (V) used in step (B) is a chiral compound wherein R2 denotes a lower alkyl group, said compound (V) being used at least predominantly in its S configuration or at least predominantly in its R configuration.

**11.** Process according to claim 10, **characterised in that** the compound (V) is used in its optically pure S form.

**12.** Process according to claim 11, **characterised in that** the compound (V) is prepared by a condensation reaction of an acrylic acid of formula (VI) with an amino ester of formula (VIII) derived from a natural amino acid.

**13.** Process according to any one of claims 10 to 12, **characterised in that** chirality inducers are used in step (B).

**14.** Process according to any one of claims 10 to 12, **characterised in that** it comprises, following step (B), a subsequent step (C) of separation of the diastereoisomers obtained in step (B).

**15.** Process according to any one of claims 1 to 9, **characterised in that** said compound of formula (I) that is obtained

is benzyl N-(RS)-[2-acetylthiomethyl-1-oxo-3-phenyl-propyl]-glycinate of formula (II) :

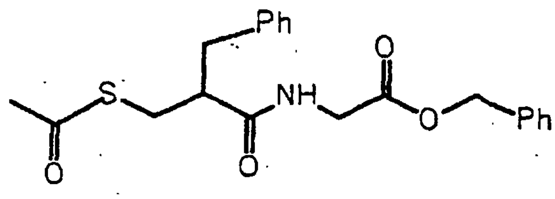

(II).

16. Process according to any one of claims 1 to 14, **characterised in that** said compound of formula (I) that is obtained is benzyl N-[S)-[2-acetylthiomethyl-1-oxo-3-(3,4-methylenedioxy-phenyl)-propyl]-(S) -alaninate of formula (III):

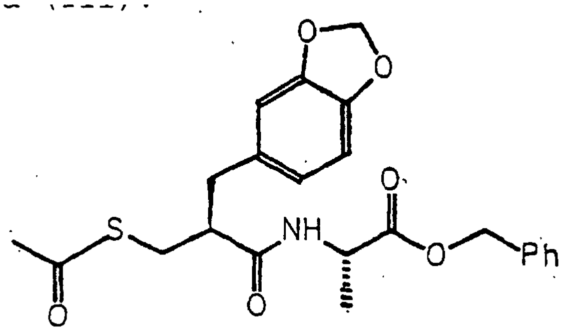

(III)